# EUROPÄISCHE PATENTSCHRIFT

(11) **EP 0 597 376 B1**
(45) Veröffentlichungstag und Bekanntmachung des Hinweises auf die Patenterteilung: **18.10.1995**
(21) Anmeldenummer: 93117816.4
(22) Anmeldetag: 03.11.1993
(51) Int. Cl.: C07C 209/90, C07C 211/52

(54) **Stabile 3,3'-Dichlorbenzidin-Dihydrochlorid-Suspension**
3,3'-Dichlorobenzidine-dihydrochloride stable suspension
Suspension de dihydrochlorure de 3,3'-dichlorobenzidine stable

(30) Priorität: 10.11.1992 DE 4237816
(43) Veröffentlichungstag der Anmeldung: 18.05.1994
(73) Patentinhaber: HOECHST AKTIENGESELLSCHAFT, 65929 Frankfurt am Main (DE)
(72) Erfinder: Steidl, Dieter, D-65719 Hofheim am Taunus (DE); Dopfer, Peter, D-65931 Frankfurt am Main (DE); Ebertz, Wolfgang, Dr.rer.net., D-65933 Frankfurt am Main (DE); Schutt, Ernst, D-64546 Mörfelden (DE)

(56) Entgegenhaltungen:
- WO-A-91/07377
- US-A- 4 559 160
- DATABASE WPI Week 8912, Derwent Publications Ltd., London, GB; AN 89-090832

## Beschreibung

Die vorliegende Erfindung betrifft eine 3,3'-Dichlorbenzidin-Dihydrochlorid-Suspension, die sich durch eine niedrige Viskosität und eine geringe Absetzrate des Feststoffes auszeichnet.

3,3'-Dichlorbenzidin ist ein technisch relevantes Ausgangsprodukt für die Herstellung von Azofarbmitteln.

Die Anforderungen an die Arbeitshygiene beim Umgang mit 3,3'-Dichlorbenzidin oder dessen Dihydrochlorid machen Maßnahmen erforderlich, die den Kontakt von Mensch und Umwelt mit diesem Stoff auf ein Minimum herabsetzen. Zur Verhinderung einer Exposition durch Stäube des 3,3'-Dichlorbenzidin-Dihydrochlorids (DCBDC) ist die Verarbeitung in Suspension das Verfahren der Wahl. Bei der Verarbeitung einer Suspension des Kristallisates der besagten Verbindung direkt aus der Produktion treten technische Probleme durch das rapide Sedimentierverhalten einer so hergestellten sogenannten "Maische" auf. Einmal sedimentiertes Dihydrochlorid läßt sich nur unter hohem Rühraufwand wieder resuspendieren. Für den Transport zu Weiterverarbeitern ist eine derartige Suspension ungeeignet.

Gemäß der US-PS 4,559,160 können stabile Suspensionen, sogenannte "slurries", des 3,3'-Dichlorbenzidin-Dihydrochlorids durch eine Naßvermahlung in verdünnten Mineralsäuren hergestellt werden. Angestrebt wird dabei eine möglichst geringe Absetzrate bei möglichst niedriger Viskosität. Die Viskosität dient als Maß für die Handhabbarkeit bezüglich Pumpbarkeit und Auslaufverhalten aus Behältern. Das Absetzverhalten ist von der Säurekonzentration und dem Feststoffgehalt abhängig. Die besagte US-Patentschriff lehrt, daß der Gehalt an DCBDC zwischen 35 und 50 Gewichtsprozent, vorzugsweise etwa 40 Gewichtsprozent, bezogen auf das freie Diamin, der Säuregehalt zwischen 1 und 10 Gewichtsprozent, vorzugsweise zwischen 1 und 5 Gewichtsprozent, sowie der Wassergehalt zwischen 40 und 50 Gewichtsprozent liegen muß, um den gestellten Anforderungen gerecht zu werden. Weiterhin geht aus Example III der besagten US-Patentschrift hervor, daß die Sedimentation von 35 bis 45 gewichtsprozentigen DCBDC-Suspensionen in der Tendenz zunimmt, wenn die Säurekonzentration schrittweise von 3 auf 10 Gewichtsprozent erhöht wird. Für den Fachmann ergibt sich aus diesen Befunden, daß sich eine Erhöhung der Säurekonzentration über 10 Gew.-% bei Suspensionen mit einem Gehalt von 35 bis 45 Gew.-% an DCBDC nachteilig auf das gewünschte Sedimentierverhalten auswirkt. Bei Suspensionen mit einem Gehalt von DCBDC über 50 Gew.-% ist der Einfluß dem Säurekonzentration auf die Sedimentation vernachlässigbar klein, jedoch sind solche konzentrierten Suspensionen sehr zähflüssig und schlecht pumpbar.

Die Aufgabe der vorliegenden Erfindung bestand darin, eine stabile DCBDC-Suspension zur Verfügung zu stellen, die eine möglichst hohe Konzentration an DCBDC und Salzsäure und dementsprechend ein möglichst geringes Volumen und geringen Wassergehalt aufweist und gleichzeitig eine möglichst niedrige Viskosität besitzt.

Es wurde gefunden, daß die Aufgabe der vorliegenden Erfindung überraschenderweise durch eine höhere Salzsäurekonzentration als im Stand der Technik beschrieben gelöst werden kann.

Gegenstand der vorliegenden Erfindung ist eine stabile 3,3'-Dichlorbenzidin-Dihydrochlorid-Suspension in Salzsäure, bestehend aus den Komponenten
a) 35 bis 63,5 Gew.-%, vorzugsweise 35 bis 55 Gew.-% aus 3,3'-Dichlorbenzidin-Dihydrochlorid;
b) 11 bis 24 Gew.-%, vorzugsweise 14 bis 19 Gew.-% aus Chlorwasserstoff und
c) mindestens 25 Gew.-% aus Wasser,
jeweils bezogen auf das Gesamtgewicht der Suspension (100 Gew.-%) und mit der Maßgabe, daß die Komponenten a), b) und c) zusammen 100 Gew.-% ergeben; und deren Viskosität im Bereich von 80 bis 1400, vorzugsweise 200 bis 800, mPa·s liegt.

Gegenstand der vorliegenden Erfindung ist auch ein Verfahren zur Herstellung der erfindungsgemäßen DCBDC-Suspension, dadurch gekennzeichnet, daß man 3,3'-Dichlorbenzidin-Dihydrochlorid mit einer 17 bis 37 gew.-%igen wäßrigen Salzsäure, vorzugsweise mit konzentrierter Salzsäure, verrührt, so daß eine Maische mit einem Feststoffgehalt an 35 bis 63,5 Gew.-% resultiert, und anschließend die Masche in einer geeigneten Apparatur naßvermahlt, bis die mittlere Korngröße (d₅₀) der DCBDC-Partikel zwischen 10 und 40 µm, vorzugsweise 15 und 25 µm, liegt.

Das zur Herstellung der Masche eingesetzte DCBDC liegt vorzugsweise in kristalliner Form vor. Unter "Maische" wird in diesem Zusammenhang eine nicht gemahlene, wäßrige DCBDC-Suspension verstanden, in der sich der Feststoff noch relativ rasch absetzt. Um eine Sedimentation so weit wie möglich zu verhindern, wird die Maische in einer geeigneten Apparatur, vorzugsweise auf einer Kolloidmühle, die nach dem Rotor-Stator-Prinzip arbeitet, oder in einer Mühle, die als Mahlwerkzeuge Mahlkörper benutzt, beispielsweise Kugel- oder Rührwerkskugelmühlen, naßvermahlen, bis die Korngröße der DCBDC-Partikel in der entstehenden Suspension in dem vorstehend angegebenen Bereich liegt.

Bei einer Vermahlung zu mittleren Korngrößen unter 10 µm steigt die Viskosität der Suspension stark an, außerdem wird thixotropes Verhalten beobachtet. Solche feingemahlenen Suspensionen sind für das erfindungsgemäße Anwendungsgebiet nicht geeignet.

Als Maß für die Sedimentation eines Feststoffes in einer Suspension dient die sogenannte Absetzrate, definiert als der Quotient aus abgesetztem Volumen und Gesamtvolumen. Zur Bestimmung dieses Quotienten füllt man ein bestimmtes Volumen einer Suspension (Gesamtvolumen) in einen Meßzylinder ein und läßt die Suspension für eine bestimmte vorgegebene Zeit, beispielsweise 24 Stunden, sedimentieren. Nach dieser Zeit wird das Volumen gemessen, das sich noch in suspendiertem Zustand befindet. Eine Aufschlämmung, worin die Partikel vollständig suspendiert sind, hat eine Absetzrate von 1,0. Für den erfindungsgemäßen Zweck sind Absetzraten von 0,9 und darüber, bei einer Standzeit von 72 Stunden, geeignet. Die Absetzraten des Feststoffes liegen beispielsweise nach 24 Stunden bei 50 gew.-%iger Suspension im Bereich zwischen 0,95 und 0,99 und bei 35 gew.-%iger Suspension im Bereich zwischen 0,92 und 0,98 sowie nach 72 Stunden im Bereich zwischen 0,90 und 0,94.

Die erfindungsgemäße Suspension läuft auch nach längerer, ruhender Lagerung problemlos aus Behältern aus. Auch bei winterlichen Temperaturen bis zu -10°C neigt die erfindungsgemäße Suspension weder zum Einfrieren noch zum Eindicken. Ein kontaminationsfreier Umgang beim Verbraucher ist somit möglich. Durch den geringeren Wassergehalt der erfindungsgemäßen Suspension im Vergleich zu den im Stand der Technik bekannten DCBDC-Suspensionen läßt sich der Transportaufwand verringern, die Raumzeitausbeute bei der Synthese der entsprechenden Azofarbmittel erhöhen und die Abwassermengen vermindern.

Die aus 3,3'-Dichlorbenzidin oder dessen Hydrochlorid hergestellten Azopigmente sind von größter Bedeutung im Sortiment der Azofarbmittel, insbesondere im Illustrationstiefdruck und Offsetdruck.

In den nachfolgenden Beispielen, die nur der Erläuterung dienen und nicht als Beschränkung der vorliegenden Erfindung anzusehen sind, bedeuten Prozente jeweils Gewichtsprozente.

### Beispiele

In einem gewogenen Rührbehälter werden Salzsäure und gegebenenfalls Wasser im gewünschten Verhältnis vorgelegt. Über eine Einfüllvorrichtung wird 3,3'-Dichlorbenzidin-Dihydrochlorid in der benötigten Menge unter Rühren zugefügt. Die so hergestellte Maische wird mittels einer drehzahlgeregelten Schlauchpumpe einer Rührwerkskugelmühle zugeführt. Nach einmaligem Durchgang durch die Mühle wird die gemahlene Suspension in einem Vorratsgefäß gesammelt.

In der nachfolgenden Tabelle 1 beträgt die Ansatzgröße jeweils 14 kg Suspension. Die Prozentangaben bedeuten Gewichtsprozente, bezogen auf das Gesamtgewicht der Suspension. Die Verweilzeiten sind Durchschnittswerte und errechnen sich aus Durchsatz und freiem Volumen der Kugelmühle.

**Tabelle 1**

| Beispiel | % HCl | % H₂O | % DCBDC | Verweilzeit in min |
|---|---|---|---|---|
| 1 | 15 | 35 | 50 | 5,2 |
| 2 | 15 | 35 | 50 | 3,5 |
| 3 | 18 | 42 | 40 | 5,6 |
| 4 | 14,5 | 31,5 | 54 | 5,6 |
| 5 | 15 | 35 | 50 | 2,4 |
| 6 | 11,7 | 38,3 | 50 | 5,5 |
| 7 | 16,4 | 33,6 | 50 | 5,4 |
| 8 | 18,6 | 31,4 | 50 | 5,5 |

In der nachfolgenden Tabelle 2 werden als Ergebnis der Versuche die Viskosität, die Absatzrate nach 24 Stunden Standzeit und die mittlere Korngröße (d₅₀) der DCBDC-Partikel gegenübergestellt.

**Tabelle 2**

| Beispiel | Viskosität in mPas | Absatzrate in ml/ml | d₅₀ in µm |
|---|---|---|---|
| 1 | 580 | 0,982 | 17 |
| 2 | 215 | 0,976 | 28 |
| 3 | 210 | 0,977 | 22 |
| 4 | 470 | 0,990 | 18 |
| 5 | 82 | 0,954 | 36 |
| 6 | 395 | 0,988 | 22 |
| 7 | 570 | 0,984 | 18 |
| 8 | 630 | 0,992 | 26 |

## Patentansprüche

1. Stabile 3,3'-Dichlorbenzidin-Dihydrochlorid-Suspension in Salzsäure, bestehend aus den Komponenten
a) 35 bis 63,5 Gew.-% aus 3,3'-Dichlorbenzidin-Dihydrochlorid;
b) 11 bis 24 Gew.-% aus Chlorwasserstoff;
c) mindestens 25 Gew.-% aus Wasser,
jeweils bezogen auf das Gesamtgewicht der Suspension (100 Gew.-%) und mit der Maßgabe, daß die Komponenten a), b) und c) zusammen 100 Gew.-% ergeben;
und deren Viskosität im Bereich von 80 bis 1400 mPa·s liegt.

2. Suspension nach Anspruch 1, dadurch gekennzeichnet, daß die Suspension zu 35 bis 55 Gew.-% aus 3,3'-Dichlorbenzidin-Dihydrochlorid, bezogen auf das Gesamtgewicht der Suspension, besteht.

3. Suspension nach Anspruch 1, dadurch gekennzeichnet, daß die Suspension zu 14 bis 19 Gew.-% aus Chlorwasserstoff, bezogen auf das Gesamtgewicht der Suspension, besteht.

4. Suspension nach Anspruch 1, dadurch gekennzeichnet, daß die Suspension zu 32 bis 45 Gew.-% aus Wasser, bezogen auf das Gesamtgewicht der Suspension, besteht.

5. Suspension nach Anspruch 1, dadurch gekennzeichnet, daß die Viskosität der Suspension im Bereich von 200 bis 800 mPa·s liegt.

6. Suspension nach mindestens einem der Ansprüche 1 bis 5, bestehend aus den Komponenten
a) 35 bis 55 Gew.-% aus 3,3'-Dichlorbenzidin-Dihydrochlorid;
b) 14 bis 19 Gew.-% aus Chlorwasserstoff;
jeweils bezogen auf das Gesamtgewicht der Suspension (100 Gew.-%) und mit der Maßgabe, daß die Komponenten a), b) und c) zusammen 100 Gew.-% ergeben; und deren Viskosität im Bereich von 200 bis 800 mPa·s liegt.

7. Suspension nach mindestens einem der Ansprüche 1 bis 6, dadurch gekennzeichnet, daß die mittlere Korngröße der Feststoffpartikel zwischen 10 und 40 µm, vorzugsweise zwischen 15 und 25 µm, liegt.

8. Verfahren zur Herstellung einer Suspension nach mindestens einem der Ansprüche 1 bis 7, dadurch gekennzeichnet, daß man 3,3'-Dichlorbenzidin-Dihydrochlorid mit einer 17 bis 37 gew.-%igen wäßrigen Salzsäure, vorzugsweise mit konzentrierter Salzsäure, verrührt, so daß eine Masche mit einem Feststoffgehalt an 35 bis 63,5 Gew.-% resultiert, und anschließend die Maische in einer geeigneten Apparatur naßvermahlt, bis die mittlere Korngroße (d₅₀) der DCBDC-Partikel zwischen 10 und 40 µm, vorzugsweise 15 und 25 µm, liegt.

9. Verfahren nach Anspruch 8, dadurch gekennzeichnet, daß die Maische auf einer Kolloidmühle, die nach dem Rotor-Stator-Prinzip arbeitet, oder in einer Kugel- oder Rührwerkskugelmühle vermahlen wird.

10. Verwendung einer Suspension nach mindestens einem der Ansprüche 1 bis 7 zur Herstellung von Azofarbmitteln.

## Claims

1. A stable 3,3'-dichlorobenzidine dihydrochloride suspension in hydrochloric acid, comprising the components
a) 35 to 63.5% by weight of 3,3'-dichlorobenzidine dihydrochloride;
b) 11 to 24% by weight of hydrogen chloride;
c) at least 25% by weight of water,
in each case relative to the total weight of the suspension (100% by weight) and with the proviso that the components a), b) and c) together make 100% by weight;
and whose viscosity is in the range from 80 to 1400 mPa·s.

2. A suspension as claimed in claim 1, wherein the suspension consists to 35 to 55% by weight of 3,3'-dichlorobenzidine dihydrochloride, relative to the total weight of the suspension.

3. A suspension as claimed in claim 1, wherein the suspension consists to 14 to 19% by weight of hydrogen chloride, relative to the total weight of the suspension.

4. A suspension as claimed in claim 1, wherein the suspension consists to 32 to 45% by weight of water, relative to the total weight of the suspension.

5. A suspension as claimed in claim 1, wherein the viscosity of the suspension is in the range from 200 to 800 mPa·s.

6. A suspension as claimed in at least one of claims 1 to 5, comprising the components
a) 35 to 55% by weight of 3,3'-dichlorobenzidine dihydrochloride;
b) 14 to 19% by weight of hydrogen chloride;
in each case relative to the total weight of the suspension (100% by weight) and with the proviso that the components a), b) and c) together make 100% by weight;
and whose viscosity is in the range from 200 to 800 mPa·s.

7. A suspension as claimed in at least one of claims 1 to 6, wherein the mean grain size of the solid particles is between 10 and 40 µm, preferably between 15 and 25 µm.

8. A process for the preparation of a suspension as claimed in at least one of claims 1 to 7, which comprises stirring 3,3'-dichlorobenzidine dihydrochloride with a 17 to 37% strength by weight aqueous hydrochloric acid, preferably with concentrated hydrochloric acid, so that a mash having a solids content of 35 to 63.5% by weight results, and then wet-grinding the mash in a suitable apparatus until the mean grain size (d₅₀) of the DCBDC particles is between 10 and 40 µm, preferably 15 and 25 µm.

9. The process as claimed in claim 8, wherein the mash is ground in a colloid mill which operates on the rotor-stator principle, or in a ball mill or stirred ball mill.

10. The use of a suspension as claimed in at least one of claims 1 to 7 for the preparation of azo colorants.

## Revendications

1. Suspension stable du dichlorhydrate de 3,3'-dichlorobenzidine dans de l'acide chlorhydrique, qui comprend :
a) de 35 à 63,5 % en poids de dichlorhydrate de 3,3'-dichlorobenzidine,
b) de 11 à 24 % en poids de chlorure d'hydrogène, et
c) au moins 25 % en poids d'eau,
toutes proportions rapportées au poids total de la suspension (100 %), à la condition que la somme des composants a), b) et c) représente 100 % en poids, et dont la viscosité est comprise entre 80 et 1400 mPa·s.

2. Suspension selon la revendication 1, caractérisée en ce qu'elle est formée pour 35 à 55 % de son poids total du dichlorhydrate de 3,3'-dichlorobenzidine.

3. Suspension selon la revendication 1, caractérisée en ce qu'elle est formée de chlorure d'hydrogène pour 14 à 19 % de son poids total.

4. Suspension selon la revendication 1, caractérisée en ce qu'elle est formée d'eau pour 32 à 45 % de son poids total.

5. Suspension selon la revendication 1, caractérisée en ce que sa viscosité est de 200 à 800 mPa·s.

6. Suspension selon une ou plusieurs des revendications 1 à 5, constituée de
a) 35 à 55 % en poids de dichlorhydrate de 3,3'-dichlorobenzidine et
b) 14 à 19 % en poids de chlorure d'hydrogène,
toutes proportions rapportées au poids total de la suspension (100 %) à la condition que les composants a) et b) représentent à eux deux 100 % en poids ; et dont la viscosité est comprise entre 200 et 800 mPa·s.

7. Suspension selon au moins une une des revendications 1 à 6, caractérisée en ce que la dimension moyenne des particules de la matière solide est comprise entre 10 et 40 µm, de préférence entre 15 et 25 µm.

8. Procédé de préparation d'une suspension selon au moins une des revendications 1 à 7, procédé caractérisé en ce que l'on délaie du dichlorhydrate de 3,3'-dichlorobenzidine avec de l'acide chlorhydrique aqueux à 17 à 37 % en poids, de préférence avec de l'acide chlorhydrique concentré, de manière à former un moût d'une teneur en matières solides de 35 à 63,5 % en poids, que l'on soumet ensuite à un broyage à l'état humide dans un appareil approprié jusqu'à ce que la dimension moyenne (d₅₀) des particules du DCBDC soit de 10 à 40 µm, de préférence de 15 à 25 µm.

9. Procédé selon la revendication 8, caractérisé en ce que l'on broie le moût dans un broyeur colloïdal, fonctionnant d'après le principe rotor-stator, ou dans un broyeur à billes ou un broyeur-agitateur à billes.

10. L'emploi d'une suspension selon au moins une des revendications 1 à 7, pour la fabrication de colorants azoïques.
